# EUROPÄISCHE PATENTSCHRIFT

(11) **EP 0 453 969 B1**
(45) Veröffentlichungstag und Bekanntmachung des Hinweises auf die Patenterteilung: **04.09.1996**
(21) Anmeldenummer: 91106268.5
(22) Anmeldetag: 18.04.1991
(51) Int. Cl.: C12N 15/62, C12N 15/76, C12N 5/10

(54) **Verfahren zur Herstellung von Fremdproteinen in Streptomyceten**
Process for production of stranger protein in streptomycetes
Procédé de production de protéine étranger en streptomycètes

(30) Priorität: 21.04.1990 DE 4012818
(43) Veröffentlichungstag der Anmeldung: 30.10.1991
(73) Patentinhaber: HOECHST AKTIENGESELLSCHAFT, 65926 Frankfurt am Main (DE)
(72) Erfinder: Koller, Klaus-Peter, Dr., W-6232 Bad Soden am Taunus (DE); Riess, Günther, Dr., W-6230 Frankfurt am Main (DE)

(56) Entgegenhaltungen:
- EP-A- 281 090
- EP-A- 289 936
- EP-A- 367 163

## Beschreibung

Aus der europäischen Patentanmeldung mit der Veröffentlichungsnummer (EP-A) 0 289 936 ist es bekannt, Fusionsproteine dadurch herzustellen, daß man das Strukturgen für das gewünschte Protein an das 3'-Ende des kodierenden Stranges des gegebenenfalls modifizierten Tendamistat-Gens koppelt, diese Genstruktur in einer Streptomyceten-Wirtszelle zur Expression bringt und das sekretierte Fusionsprotein aus dem Überstand isoliert. In einer bevorzugten Ausführungsform wird das Tendamistat-Gen am 3'-Ende verkürzt. Für die Verkürzung werden die Schnittstellen für die Restriktionsenzyme BstEII im Bereich der Tripletts 31 und 32, StuI im Bereich der Tripletts 43 und 44 sowie Sau3A im Bereich der Tripletts 52 und 53 verwendet.

In Weiterentwicklung dieses Erfindungsgedankens wurde bereits vorgeschlagen, ein Fusionsprotein herzustellen, in dem auf den Tendamistat-Anteil ein verkürztes Proinsulin folgt, dessen C-Kette lediglich aus einem oder zwei Lysin-Resten besteht ("Mini-Proinsulin"). Als weitere Fortentwicklung wurde vorgeschlagen, daß in derartigen Fusionsproteinen auch der Tendamistat-Anteil verkürzt ist (am 9.5.1990 veröffentlichte EP-A 0 367 163).

Es wurde nun überraschenderweise gefunden, daß Fusionsproteine mit einem sehr kurzen Tendamistat-Anteil in der Streptomycetenzelle stabil sind und in das Medium ausgeschleust werden. Die so erhaltenen Fusionsproteine verhalten sich aufgrund der sehr kurzen Tendamistat-Kette wie "reife" Proteine und liegen im allgemeinen in der richtigen Tertiärstruktur im Medium vor.

Aus der EP-A 0 177 827 ist eine synthetische Signalsequenz zum Transport von Proteinen in Expressionssystemen bekannt, die dadurch gekennzeichnet ist, daß die DNA im wesentlichen einer natürlichen Signalsequenz entspricht, aber eine oder mehrere Schnittstellen für Endonukleasen aufweist, die in der natürlichen DNA nicht enthalten sind. Wenn man das Gen für das zu transportierende Protein an eine solche DNA-Sequenz koppelt, dieses Fusionsgen in einen Vektor einbaut und damit eine Wirtszelle transformiert, die das exprimierte Protein aus dem Cytoplasma transportiert, kann man eukaryotische, prokaryotische oder virale Proteine in prokaryotischen und eukaryotischen Zellen herstellen. Am Beispiel des periplasmatischen Proteins Alkalische Phosphatase wird gezeigt, daß es bei der Expression in E. coli vorteilhaft ist, im Anschluß an die Prä-Sequenz die Codons für die etwa ersten 40 Aminosäuren der Alkalischen Phosphatase vor das Strukturgen für das gewünschte Protein zu setzen. In vielen Fällen genügen jedoch auch weniger zusätzliche Aminosäuren, beispielsweise etwa 10, vorteilhaft etwa 5. Ein entsprechendes Fusionsprotein mit Affen-Proinsulin wurde zu etwa 90 % in den periplasmatischen Raum transportiert.

Es wurde auch schon vorgeschlagen (WO 91/03550, veröffentlicht am 21.3.1991), Fusionsproteine dadurch herzustellen, daß man ein gemischtes Oligonukleotid konstruiert, das für den Ballast-Anteil des Fusionsproteins kodiert, dieses Oligonukleotid so in einen Vektor einbringt, daß es funktionell an eine Regulationsregion und das Strukturgen für das gewünschte Protein gekoppelt ist, mit dieser so erhaltenen Plasmidpopulation geeignete Wirtszellen transformiert und diejenigen Klone, die eine hohe Ausbeute an kodiertem Fusionsprotein zeigen, selektiert.

Das Oligonukleotid besteht hierbei vorzugsweise aus 4 bis 12, insbesondere 4 bis 8 Tripletts.

Es wurde schon versucht, Fusionsproteine mit einem kurzen Ballast-Anteil herzustellen. So wurde beispielsweise eine Genfusion hergestellt, die für ein Fusionsprotein aus den ersten 10 Aminosäuren der ß-Galactosidase und Somatostatin kodiert. Es zeigte sich jedoch, daß dieses kurze ß-Galactosidasefragment nicht ausreichte, um das Fusionsprotein vor dem Abbau durch die wirtseigenen Proteasen zu schützen (US-A 4 366 246, Spalte 15, Absatz 2). Dementsprechend wurden in den EP-A 0 290 005 und 0 292 763 Fusionsproteine beschrieben, deren Ballast-Anteil aus einem ß-Galactosidasefragment mit mehr als 250 Aminosäuren besteht.

Unerwarteterweise sind nun aber Fusionsproteine aus den ersten sieben bis zehn aminoterminalen Aminosäuren von Tendamistat und einem gewünschten Protein, beispielsweise einem Proinsulin, in Streptomyceten-Wirtszellen stabil und werden in das Medium sekretiert, aus dem sie in hohen Ausbeuten gewonnen werden können. Überraschenderweise gilt das auch für relativ kleine Proteine wie "Mini-Proinsuline".

Fusionsproteine, in deren Tendamistatanteil in Position 7 und/oder 9 Prolin (wie in der natürlichen Sequenz) steht, sind bevorzugt.
Selbstverständlich ist es aber möglich, entsprechend den bereits bekannten oder vorgeschlagenen Ausführungsformen (EP-A 0 367 163) einen größen Tendamistat-Ballastanteil zu wählen, wobei natürlich der Vorteil des geringen "Ballastes" mehr und mehr verloren geht.

Es ist möglich und sogar vorteilhaft, die natürliche Aminosäurefolge im Bereich der siebten und bis zur zehnten Aminosäure des Tendamistat-Anteils durch Aminosäureaustausche zu verändern. Weiterhin ist es möglich, die Aminosäurefolge im Signalpeptid zu variieren.

Besonders günstige Fusionskonstruktionen können in Kenntnis des Erfindungsgedankens durch einfache Vorversuche leicht ermittelt werden.

Weiterhin ist es möglich, den Erfindungsgedanken auch in anderen Gram-positiven Bakterienzellen zu verwirklichen, beispielsweise in Bacillus- oder Staphyllococcus-Zellen unter Verwendung von Signalsequenzen, die von diesen Wirten "erkannt" werden.

Die erfindungsgemäß erhaltenen Fusionsproteine liegen im Medium in gelöster Form vor, was viele Vorteile bei der Weiterverarbeitung und Reinigung bietet. So kann beispielsweise eine enzymatische Weiterverarbeitung unter Abspaltung des Ballastanteils unmittelbar mit dem Sekretionsprodukt erfolgen, ohne daß Aufarbeitungsschritte unternommen werden müßten, wie sie bei unlöslichen Fusionsproteinen erforderlich sind. Es kann auch vor der Weiterverarbeitung erst eine Aufkonzentration oder Reinigung, beispielsweise durch Affinitätschromatographie, aber auch Ultrafiltration, Fällung, lonenaustauschchromatographie, Adsorptionschromatographie, Gelfiltration oder Hochdruckflüssigkeitschromatographie erfolgen.

In den folgenden Beispielen wird die Erfindung näher erläutert.

Das Ausgangsmaterial für die Plasmidkonstruktionen ist das Plasmid pKKSOO, das in der EP-A 0 367 163 vorgeschlagen wurde. Dieses Plasmid unterscheidet sich von dem aus der EP-A 0 289 936 bekannten Plasmid pKK400 durch den Ersatz des Proinsulingens durch ein analoges Gen, das an Stelle der C-Kette lediglich die Aminosäure Lysin codiert, sowie durch die Einfügung einer Terminator-Sequenz im Anschluß an dieses "Mini-Proinsulin"-Gen. Die Tabellen 1 und 2 aus der EP-A 0 367 163, in denen das "Mini-Proinsulin"-Gen bzw. die Terminator-Sequenz wiedergegeben sind, sind als Anhang der Beschreibung beigefügt.

Die Plasmide pKK400 und pKK500 enthalten in der Signalsequenz des α-Amylaseinhibitor-Gens eine XmaIII-Schnittstelle (im Bereich der Tripletts -5 bis -7).

### Beispiel 1

Das Plasmid pKK500 wird mit den Restriktionsenzymen EcoRI und XmaIII geöffnet und das große Fragment auf einem 0,8 %igen Agarosegel durch Gelelektrophorese getrennt und durch Elektroelution isoliert. Dieses Fragment wird mit dem nach der Phosphoramidit-Methode synthetisierten DNA-Fragment (1) (SEQ ID NO:1) 
          -5                -1
     Ala Gly Pro Ala Ser Ala
5' G GCC GGG CCG GCC TCC GCC
3'       CCC GGC CGG AGG CGG
   (XmaIII)

Asp Thr Thr Val Ser Glu Pro
GAC ACG ACC GTC TCC GAG CCG       3'
CTG TGC TGG CAG AGG CTC GGC TTA A 5'
                             (EcoRI)
 ligiert und das Ligationsgemisch in E. coli transformiert. Man erhält das Plasmid pKK510. Dieses codiert ein Präproinsulin, in dem auf die Signalsequenz des Tendamistat die ersten 7 Aminosäuren von Tendamistat und hierauf die Mini-Proinsulinkette folgen.

### Beispiel 2

Analog dem in der EP-A 0 289 936 beschriebenen Verfahren zur Überführung des Plasmids pKK400 in das Expressionsplasmid pGF1 wird das Plasmid pKK510 in das Expressionsplasmid pKF1 überführt:

Die isolierte Plasmid-DNA von pKK510 wird mit den Restriktionsenzymen SphI und SstI geschnitten und das kleine Fragment mit dem Fusionsgen isoliert. Das handelsübliche Expressionsplasmid pIJ 702 (erhältlich bei der John Innes Foundation, Norwich, England) wird mit den gleichen Enzymen geschnitten und das große Fragment isoliert. Diese beiden isolierten Fragmente werden ligiert, das Ligationsgemisch in S. lividans TK24 transformiert und aus den Thiostrepton-resistenten, weißen (d.h. nicht zur Melaninbildung befähigten) Transformanten das Plasmid isoliert. Klone, die das eingesetzte Insert tragen, werden auf ihre Bildung von Fusionsproteinen in der Schüttelkultur untersucht.

Die Expression des kodierten Fusionsproteins erfolgt in an sich bekannter Weise: Inkubiert man den transformierten Stamm 4 Tage über 25°C im Schüttelkolben und trennt das Mycel von der Kulturlösung durch Zentrifugieren ab, so kann man das gebildete Fusionsprotein im Kulturüberstand nach Elektrophorese von 20 µm Kulturfiltrat in einem 15 %igem Polyacrylamidgel durch Anfärbung mit ®COOMASSIE-Blau als zusätzliche Proteinbande sichtbar machen, die in einem Kontrollexperiment nicht auftritt, in den der Stamm nur mit pIJ 702 transformiert wurde.

Behandelt man das Kulturfiltrat mit Lysylendoproteinase, so kann man gelelektrophoretisch Des-(B30)-Thr-Insulin nachweisen, das durch eine authentische Kontrolle verifiziert wird.

Weiterhin kann man im Kulturfiltrat das Fusionsprotein mit Insulin-Antikörpern entweder im Immuno-Blot oder mit einem Insulin-RIA nachweisen.

### Beispiel 3

Man verfährt gemäß Beispiel 1 und 2, setzt jedoch das synthetische Fragment (2) mitder SEQ ID NO:2 
-5                -1
Ala Gly Pro Ala Ser Ala
5' G GCC GGG CCG GCC TCC GCC
3'       CCC GGC CGG AGG CGG
(XmaIII)

5
Asp Thr Thr Val Ser Glu Pro Asp Pro
GAC ACG ACC GTC TCC GAG CCC GAC CCG          3'
CTG TGC TGG CAG AGG CTC GGG CTG GGC TTA A    5'
(EcoRI)
 ein, so erhält man die Plasmide pKK320 bzw. pKF2.

Diese Plasmide kodieren ein Fusionsprotein, das sich von dem gemäß Beispiel 1 und 2 dadurch unterscheidet, daß auf die ersten 7 Aminosäuren des Tendamistat Asparagin (anstelle der natürlichen Aminosäure Alanin) und hierauf die neunte Aminosäure im Tendamistat, Prolin, folgen. Durch den Austausch von Alanin gegen Asparagin wird also eine zusätzliche positive Ladung in den Ballastanteil des Fusionsproteins eingeführt. Überraschenderweise erhält man etwa 20 bis 30 % höhere Ausbeuten als nach Beispiel 2.

### Beispiel 4

Verfährt man gemäß Beispiel 1 und 2, setzt jedoch das synthetische Fragment (3) mit der SEQ ID NO:3 
        -5              -1
     Ala Gly Pro Ala Ser Ala
5' G GCC GGG CCG GCC TCC GCC
3'       CCC GGC CGG AGG CGG
   (XmaIII)

                    5
   Asp Thr Thr Val Ser Glu Pro Ala Pro
   GAC ACG ACC GTC TCC GAG CCC GCA CCG         3'
   CTG TGC TGG CAG AGG CTC GGG CGT GGC TTA A   5'
 ein, so erhält man die Plasmide pKK330 bzw. pKF3. Diese Plasmide unterscheiden sich von denen nach Beispiel 1 und 2 dadurch, daß sie die ersten 9 natürlichen Aminosäuren des Tendamistat kodieren. Im Vergleich zu Beispiel 2 werden etwa 10 % höhere Ausbeuten erhalten.

### Beispiel 5

Das von pKK500 kodierte Fusionsprotein enthält zwischen dem Tendamistat-Anteil und der B-Kette des Proinsulins eine Linkersequenz, die für die Aminosäuren Asn-Ser-Asn-Gly-Lys kodiert. Der Ersatz dieses endständigen Lys und des die C-Kette darstellenden Lys durch Arg erfolgt wie nachstehend beschrieben. Hierbei wird von der singulären StyI-Schnittstelle im Bereich der Kodons B30 bis A1 in der Proinsulinsequenz Gebrauch gemacht.

Isolierte Plasmid-DNA von pKK500 wird mit StyI geschnitten, mit S1 Nuklease zur Entfernung der überstehenden Enden verdaut und die überchüssige Nuklease mit Phenol-Chloroform extrahiert. Das linearisierte Plasmid wird anschließend mit EcoRI nachgeschnitten, das große Fragment elektrophoretisch abgetrennt und durch Elektroelution isoliert. Dieses Fragment wird mit dem synthetischen Fragment (4) (SEQ ID NO:4) 
B1 10
Asn Ser Asn Gly Arg Phe Val Asn Gln His Leu Cys Gly Ser His
AAT TCG AAC GGC CGC TTC GTC AAC CAG CAC CTG TGC GGC TCG CAC
GC TTG CCG GCG AAG CAG TTG GTC GTG GAC ACG CCG AGC GTG
(EcoRI)

20 30
Leu Val Glu Ala Leu Tyr Leu Val Cys Gly Glu Arg Gly Phe Phe
CTC GTG GAG GCC CTC TAC CTG GTG TGC GGG GAG CGC GGC TTC TTC
GAG CAC CTC CGG GAG ATG GAC CAC ACG CCC CTC GCG CCG AAG AAG

B30 C(B31)
Tyr Thr Pro Lys Thr Arg
TAC ACC CCC AAG ACC CGC
ATG TGG GGG TTC TGG GCG
 ligiert und das Ligationsgemisch in E. coli transformiert. Die gewünschten Klone werden durch Restriktionsanalyse des enthaltenden Plasmids überprüft, wobei von der neu entstandenen SstII-Schnittstelle Gebrauch gemacht wird. Weiterhin wird das gesamte SphI-SstI-Fragment sequenziert.

Zur Expression des kodierten Fusionsproteins wird das durch Sequenzanalyse überprüfte Fragment in den mit den gleichen Enzymen geschnittenen Vektor pIJ 702 ligiert, wobei der Expressionsvektor pGF4 entsteht.

Der Nachweis des von pGF4 kodierten und sekretierten Fusionsproteins kann einerseits über den α-Amylaseinhibitor-Plattentest (EP A 0 161 629, Beispiel 3) und andererseits aus dem Überstand der Schüttelkultur analog Beispiel 2 erfolgen.

### Beispiel 6

Fügt man analog Beispiel 5 das Fragment (4) in die Vektoren pKK510, 520 und 530 ein, so erhält man die Vektoren pKK610, 620 und 630. Der Einbau der jeweiligen SphI-SstI-Fragmente mit der kodierenden Sequenz für die Fusionsproteine in den Vektor pIJ 702 ergibt die Expressionsvektoren pKF11, 12 und 13. Die Expression der sekretierten Fusionsproteine wird gemäß Beispiel 2 überprüft.

### Beispiel 7

Zur Erhöhung der Expression von Derivaten des Plasmids pIJ 702 wird aus diesem der Melaninpromotor durch Verdauung mit PstI und SphI entfernt und durch das synthetische Fragment (5) (SEQ ID NO:5) 
PstI BclI
10 20 30 40 50
CTGCAGTGATCAGGGGGACCCTTGTGCGAATTTCCGTTACGGGTTTGGGTGGTAGGG
GACGTCACTAGTCCCCCTGGGAACACGCTTAAAGGCAATGCCCAAACCCACCATCCC

SphI
60 70 80
ACGCACCCGAAGAGGAGGCCCCAGCATGC
TGCGTGGGCTTCTCCTCCGGGGTCGTACG
 ersetzt. Hierdurch ergibt sich eine Tandem-Konstruktion aus dem synthetischen und dem Tendamistat-Promotor. Das Plasmid erhält die Bezeichnung pGR110.

Werden in pGR110 nach Schneiden mit SphI und SstI die synthetischen Fragmente (1), (2) und (3) eingesetzt, so resultieren die Expressionsvektoren pGR200, 210 und 220. Analog erhält man mit dem Fragment (4) die Expressionsvektoren pGR250, 260 und 270.

### Beispiel 8

Soll aus den Insulin-Vorläufern Humaninsulin durch Kombination von Trypsin oder einem gleichwirkenden Enzym und Carboxypeptidase B hergestellt werden, ist es vorteilhaft, im Verlauf der Spaltungsreaktion rasch den aninoterminalen Ballastanteil abzuspalten, um die Spaltungsreaktion in Richtung auf das B31 (Arg)-Insulin zu begünstigen. Dazu bietet sich eine Modifikation der Aminosäuren vor der Aminosäure B1 (Phe) an:

Man verfährt gemäß Beispiel 1 und öffnet das Plasmid pKK 500 mit den Restriktionsenzymen EcoRI und DraIII. Das ursprüngliche Fragment wird dann durch das mit der Phosphoramidit-Methode synthetisierte DNA-Fragment (6) (SEQ ID NO:6) 
B1
Asn Ser Ala Arg Phe Val Asn Gln His Leu Cys Gly Ser His Leu
5' AAT TCG GCC CGC TTC GTC AAC CAG CAC CTG TGC GGC TCG CAC CTC 3'
3'      GC CGG GCG AAG CAG TTG GTC GTG GAC ACG CCG AGC GTG     5'

(EcoRI)                                                    (DraIII)
 ersetzt. Klonierung in E. coli und Expression in Streptomyces lividans erfolgen gemäß Beispiel 1 bzw. Beispiel 2. Es resultieren das Plasmid pKK640 bzw. das Expressionsplasmid pKF14.

Analog kann auch mit dem Plasmid verfahren werden, das gemäß Beispiel 5 (nach Einbau des Fragments (4)) erhalten wird. Man erhält so die Plasmide pKK650 bzw. pKF15. Anhang (aus EP-A-0 367 163):

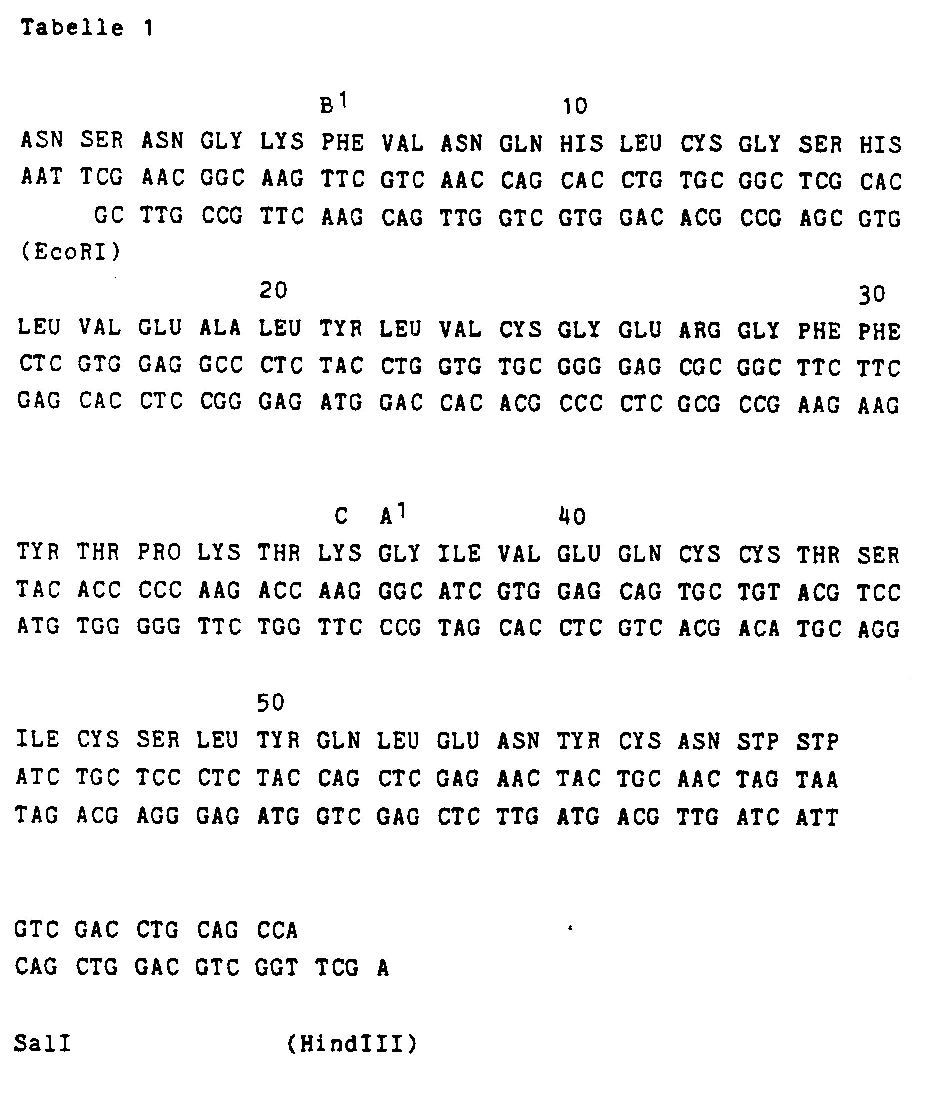

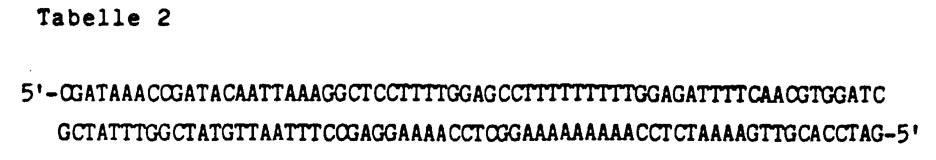

SEQ ID NO:1
- ART DER SEQUENZ:: Nucleotid mit entsprechendem Protein
- SEQUENZLÄNGE:: 44 Basen
- STRANGFORM:: Doppelstrang mit überstehenden Erkennungssequenzen
- TOPOLOGIE:: linear
- ART DES FRAGMENTS:: inneres Fragment
- HERKUNFT:: synthetische DNA 
G GCC GGG CCG GCC TCC GCC GAC ACG ACC GTC TCC GAG CCG
CCC GCG CGG AGG CGG CTG TGC TGG CAG AGG CTC GGC TTA A
Ala Gly Pro Ala Ser Ala Asp Thr Thr Val Ser Glu Pro Asn
-5 1 5
SEQ ID NO:2
- ART DER SEQUENZ:: Nucleotid mit entsprechendem Protein
- SEQUENZLÄNGE:: 50 Basen
- STRANGFORM:: Doppelstrang mit überstehenden Erkennungssequenzen
- TOPOLOGIE:: linear
- ART DES FRAGMENTS:: inneres Fragment
- HERKUNFT:: synthetische DNA 
G GCC GGG CCG GCC TCC GCC GAC ACG ACC GTC TCC GAG CCC GAC CCG
CCC GGC CGG AGG CGG CTG TGC TGG CAG AGG CTC GGG CTG GCC
Ala Gly Pro Ala Ser Ala Asp Thr Thr Val Ser Glu Pro Asp Pro
-5 1 5

TTA A
Asn
10
SEQ ID NO:3
- ART DER SEQUENZ:: Nucleotid mit entsprechendem Protein
- SEQUENZLÄNGE:: 50 Basen
- STRANGFORM:: Doppelstrang mit überstehenden Erkennungssequenzen
- TOPOLOGIE:: linear
- ART DES FRAGMENTS:: inneres Fragment
- HERKUNFT:: synthetische DNA 
G GCC GGG CCG GCC TCC GCC GAC ACG ACC GTC TCC GAG CCC GCA CCG
CCC GGC CGG AGG CGG CTG TGC TGG CAG AGG CTC GGG CGT GGC TTA A
Ala Gly Pro Ala Ser Ala Asp Thr Thr Val Ser Glu Pro Ala Pro Asn
-5 1 5
SEQ ID NO:4
- ART DER SEQUENZ:: Nucleotid mit entsprechendem Protein
- SEQUENZLÄNGE:: 108 Basen
- STRANGFORM:: Doppelstrang mit überstehenden Erkennungssequenzen
- TOPOLOGIE:: linear
- ART DES FRAGMENTS:: inneres Fragment
- HERKUNFT:: synthetische DNA 
AAT TCG AAC GGC CGC TTC GTC AAC CAG CAC CTG TGC GGC TCG CAC
    GC TTG CCG GCG AAG CAG TTG GTC GTG GAC ACG CCG AGC GTG
Asn Ser Asn Gly Arg Phe Val Asn Gln His Leu Cys Gly Ser His
                5                   10                  15

CTC GTG GAG GCC CTC TAC CTG GTG TGC GGG GAG CGC GGC TTC TTC
GAG CAC CTC CGG GAG ATG GAC CAC ACG CCC CTC GCG CCG AAG AAG
Leu Val Glu Ala Leu Tyr Leu Val Cys Gly Glu Arg Gly Phe Phe
                20                  25                  30

TAC ACC CCC AAG ACC CGC
ATG TGG GGG TTC TGG GCG
Tyr Thr Pro Lys Thr Arg
                    35
SEQ ID NO:5
- ART DER SEQUENZ:: Nucleotid mit
- SEQUENZLÄNGE:: 86 Basen
- STRANGFORM:: Doppelstrang
- TOPOLOGIE:: linear
- ART DES FRAGMENTS:: inneres Fragment
- HERKUNFT:: synthetische DNA 
CTGCAGTGAT CAGGGGGACC CTTGTGCGAA TTTCCGTTAC GGGTTTGGGT GGTAGGGACG 60

CACCCGAAGA GGAGGCCCCA GCATGC 86
SEQ ID NO:6
- ART DER SEQUENZ:: Nucleotid mit entsprechendem Protein
- SEQUENZLÄNGE:: 45 Basen
- STRANGFORM:: Doppelstrang mit überstehenden Erkennungssequenzen
- TOPOLOGIE:: linear
- ART DES FRAGMENTS:: inneres Fragment
- HERKUNFT:: synthetische DNA

AAT TCG GCC CGC TTC GTC AAC CAG CAC CTG TGC GGC TCG CAC CTC
    GC CGG GCG AAG CAG TTG GTC GTG GAC ACG CCG AGC GTG
Asn Ser Ala Arg Phe Val Asn Gln His Leu Cys Gly Ser His Leu
                5                   10                  15

## Patentansprüche (Patentansprüche für folgende(n) Vertragsstaat(en): AT, BE, CH, DE, DK, FR, GB, GR, IT, LI, LU, NL, SE)

1. Verfahren zur Herstellung von Fusionsproteinen, dadurch gekennzeichnet, daß man das Strukturgen für das gewünschte Protein an die Kodons für die Signalsequenz und die ersten sieben bis zehn aminoterminalen Aminosäuren von Tendamistat koppelt, diese Genstruktur in einer Streptomyceten-Wirtszelle zur Expression bringt und das sekretierte Fusionsprotein aus dem Überstand isoliert, wobei die Tendamistatgensequenzen im Bereich der siebten bis zur zehnten Aminosäure durch Aminosäureaustausche modifiziert sein können.

2. Genstruktur, bei der die Signalsequenz und die ersten sieben bis zehn Kodons für Tendamistat an das Strukturgen für ein anderes Protein gekoppelt sind.

3. Genstruktur nach Anspruch 2, dadurch gekennzeichnet, daß im Tendamistat-Anteil Kodons für Aminosäuren im Bereich der siebten bis zur zehnten Aminosäure ausgetauscht werden.

4. Genstruktur nach Anspruch 2 oder 3, dadurch gekennzeichnet, daß zwischem dem Tendamistatgen und dem Strukturgen für das gewünschte Protein ein DNA-Fragment angeordnet ist, welches für einen peptidischen Linker mit der Sequenz Asn-Ser-Asn-Gly-Arg kodiert.

5. Vektor, in den eine Genstruktur nach Anspruch 2, 3 oder 4 ligiert ist.

6. Streptomycetenzelle, enthaltend einen Vektor nach Anspruch 5.

7. Fusionsprotein, gekennzeichnet durch einen N-ständigen Anteil der ersten sieben bis zehn Aminosäuren von Tendamistat, gekoppelt - gegebenenfalls über eine Brückensequenz - an das gewünschte Protein.

8. Verwendung des Fusionsproteins nach Anspruch 7 bzw. des nach Anspruch 1 erhältlichen Fusionsproteins zur Herstellung des gewünschten Proteins.

## Patentansprüche (Patentansprüche für folgende(n) Vertragsstaat(en): ES)

1. Verfahren zur Herstellung von Fusionsproteinen, dadurch gekennzeichnet, daß man das Strukturgen für das gewünschte Protein an die Kodons für die Signalsequenz und die ersten sieben bis zehn aminoterminalen Aminosäuren von Tendamistat koppelt, diese Genstruktur in einer Streptomyceten-Wirtszelle zur Expression bringt und das sekretierte Fusionsprotein aus dem Überstand isoliert, wobei die Tendamistatgensequenzen im Bereich der siebten bis zur zehnten Aminosäure durch Aminosäureaustausche modifiziert sein können.

2. Verfahren zur Herstellung einer Genstruktur, dadurch gekennzeichnet, daß man die Signalsequenz und die ersten sieben bis zehn Kodons für Tendamistat an das Strukturgen für ein anderes Protein koppelt.

3. Genstruktur nach Anspruch 2, dadurch gekennzeichnet, daß im Tendamistat-Anteil Kodons für Aminosäuren im Bereich der siebten bis zur zehnten Aminosäure ausgetauscht werden.

## Claims (Claims for the following Contracting State(s): AT, BE, CH, DE, DK, FR, GB, GR, IT, LI, LU, NL, SE)

1. A process for the production of fusion proteins, which comprises coupling the structural gene for the desired protein to the codons for the signal sequence and the first seven to ten amino-terminal amino acids of tendamistat, expressing this gene structure in a streptomyces host cell and isolating the secreted fusion protein from the supernatant, it being possible for the tendamistat gene sequences to be modified in the region of the seventh to the tenth amino acid by amino acid exchanges.

2. A gene structure in which the signal sequence and the first seven to ten codons for tendamistat are coupled to the structural gene for another protein.

3. The gene structure as claimed in claim 2, wherein codons for amino acids in the region of the seventh to the tenth amino acid are exchanged in the tendamistat portion.

4. The gene structure as claimed in claim 2 or 3, wherein a DNA fragment which codes for a peptide linker having the sequence Asn-Ser-Asn-Gly-Arg is arranged between the tendamistat gene and the structural gene for the desired protein.

5. A vector into which a gene structure as claimed in claim 2, 3 or 4 is ligated.

6. A streptomyces cell containing a vector as claimed in claim 5.

7. A fusion protein comprising an N-terminal portion of the first seven to ten amino acids of tendamistat coupled - if appropriate via a bridge sequence - to the desired protein.

8. Use of the fusion protein as claimed in claim 7 or the fusion protein available according to the process as claimed in claim 1 for the production of the desired protein.

## Claims (Claims for the following Contracting State(s): ES)

1. A process for the production of fusion proteins, which comprises coupling the structural gene for the desired protein to the codons for the signal sequence and the first seven to ten amino-terminal amino acids of tendamistat, expressing this gene structure in a streptomyces host cell and isolating the secreted fusion protein from the supernatant, it being possible for the tendamistat gene sequences to be modified in the region of the seventh to the tenth amino acid by amino acid exchanges.

2. A process for the production of a gene structure, which comprises coupling the signal sequence and the first seven to ten codons for tendamistat to the structural gene for another protein.

3. The gene structure as claimed in claim 2, wherein codons for amino acids in the region of the seventh to the tenth amino acid are exchanged in the tendamistat portion.

## Revendications (Revendications pour l'(les) Etat(s) contractant(s) suivant(s): AT, BE, CH, DE, DK, FR, GB, GR, IT, LI, LU, NL, SE)

1. Procédé pour la fabrication de protéines de fusion, caractérisé en ce que l'on couple le gène de structure pour la protéine souhaitée aux codons de la séquence signal et des sept à dix premiers acides aminés amino-terminaux du Tendamistat, on exprime cette structure génique dans une cellule hôte de streptomycète et on isole du milieu la protéine de fusion sécrétée, les séquences géniques du Tendamistat pouvant être modifiées dans le domaine compris entre le septième et le dixième acide aminé par échange d'acide aminé.

2. Structure génique dans laquelle la séquence de signal et les sept à dix premiers codons du Tendamistat sont couplés au gène de structure d'une autre protéine.

3. Structure génique selon la revendication 2, caractérisée en ce que, dans la partie Tendamistat, on échange les codons pour les acides aminés dans le domaine du septième au dixième acide aminé.

4. Structure génique selon la revendication 2 ou 3, caractérisée en ce que, entre le gène du Tendamistat et le gène de structure pour la protéine souhaitée est disposé un fragment d'ADN qui code pour un linker peptidique de séquence Asn-Ser-Asn-Gly-Arg.

5. Vecteur dans lequel est ligaturé une structure génique selon la revendication 2, 3 ou 4.

6. Cellule de streptomycète, contenant un vecteur selon la revendication 5.

7. Protéine de fusion, caractérisée par la partie N-terminale des 7 à 10 premiers acides aminés du Tendamistat, couplée, éventuellement par une séquence de pontage, à la protéine souhaitée.

8. Utilisation de la protéine de fusion selon la revendlcation 7 ou selon revendication 1, respectivement, à la production de la protéine suohaitée.

## Revendications (Revendications pour l'(les) Etat(s) contractant(s) suivant(s): ES)

1. Procédé de fabrication de protéines de fusion, caractérisé en ce que l'on couple le gène de structure pour la protéine souhaitée aux codons de la séquence signal et des sept à dix premiers acides aminés amino-terminaux du Tendamistat, on exprime cette structure génique dans une cellule hôte de streptomycète et on isole du milieu la protéine de fusion sécrétée, les séquences géniques du Tendamistat pouvant être modifiées dans le domaine compris entre le septième et le dixième acide aminé par échange d'acide aminé.

2. Procédé de fabrication d'une structure génique, caractérisé en ce que l'on couple la séquence de signal et les sept à dix premiers codons du Tendamistat au gène de structure d'une autre protéine.

3. Structure génique selon la revendication 2, caractérisée en ce que, dans la partie Tendamistat, on échange les codons des acides aminés dans le domaine entre le septième et le dixième acide aminé.
